# EUROPEAN PATENT SPECIFICATION

(11) **EP 4 395 661 B1**
(45) Date of publication and mention of the grant of the patent: **11.03.2026**
(21) Application number: 22865208.7
(22) Date of filing: 01.09.2022
(51) Int. Cl.: A61B 17/06, D02J 13/00, A61B 17/00

(54) **A CUTTING AND HARDENING MACHINE FOR SURGICAL THREADS**
SCHNEID- UND HÄRTUNGSMASCHINE FÜR CHIRURGISCHE FÄDEN
MACHINE DE COUPE ET DE DURCISSEMENT POUR FILS CHIRURGICAUX

(30) Priority: 01.09.2021 TR 202113728
(43) Date of publication of application: 10.07.2024
(73) Proprietor: Tovka, Davut, Bodrum/Mugla (TR)
(72) Inventor: ALKAN, Abdullah, Menemen/Izmir (TR); KADER, Yetkin, Bayrakli/Izmir (TR)
(74) Representative: Kaya, Erdem
(86) International application number: PCT/TR2022/050933
(87) International publication number: WO 2023/033773

(56) References cited:
- FR-A1- 2 526 453
- US-A- 5 156 788
- US-A- 5 250 247
- US-A- 5 569 302
- US-A- 5 672 375
- US-B2- 8 222 564

## Description

### TECHNICAL FIELD

The invention relates to a machine (M) which provides hardening and cutting of surgical threads and prepares the thread end to be placed on the suture needle in order to prevent fraying. The machine comprises a heater/cutter mechanism (30) including a cutter mechanism (310) and a heating unit (50); a winding mechanism (20) which is aligned with the heater/cutter mechanism (30) on at least one axis; a drum (21) positioned on the winding mechanism (20) and rotatable on its axis to allow the thread to be wound thereon; a lower reflector (51), consisting of at least one part, arranged on the drum (21); and at least one cutter (31), positioned on the cutter mechanism (310), which enables the threads wound on the drum (21) to be cut.

### BACKGROUND

In surgeries, tissue incisions are attached to each other using special threads, and healing is ensured in this way. Alternative types of threads and needles are used based on the type and region of the surgery, and these threads are presented sterile for the use of the surgeon.

During the surgery or suture procedures of the incisions, the procedure is performed by holding the suture needles with the suture holder, and the suture needle ready to be packaged during the surgery or operation is removed by directly holding with the suture holder and the procedure is continued. It is important that this operation is fast and comfortable, especially when the surgical condition is considered. For this reason, suture threads are cut in the prescribed sizes and presented as connected to suture needles.

Surgical threads are generally made of synthetic material and are produced as monofilament or multifilament. Due to their structure, multifilament threads have frayed ends. Particularly in the case of cutting, the end parts are scattered, which prevents the suture needle from entering the end part. The entire end of the thread must be inside the needle in order to avoid any problems, especially during the operation.

In order to solve the said problem, the surgical threads are cut into the desired sizes in the art, the ends are hardened and then fixed by placing them in the suture needle. In known applications, multiple or linear single cutting techniques can be applied, and then the ends of the threads can be hardened by alternative methods. One of them is the hardening process carried out by using biocompatible adhesive and the other is the hardening process carried out by applying heat. In heat applied methods, direct heat is applied to the end of the thread from the heat source and the end of the thread is hardened.

In heat applications, the end of the threads are kept between the heating plates, and the threads are taken from this section after the optimum time. The heat transfer method used in this method is heat transfer. Although more stable results are obtained compared to adhesion, it causes low-capacity production in mass production, especially in multiple hardening processes. Multiple surgical thread coils are required to be used in the same main for capacity increase. Since the coil life of absorbable threads is limited, operating more than one coil at the same time can cause problems in low quantity productions and cause an increase in the waste rate. Since the heater plates operate continuously, they also heat the environment. Since surgical thread production is carried out under clean room conditions, factors that increase the ambient temperature are a negative factor for the production site. For this reason, the adhesive application is more commonly used in high capacity demands.

USPTO patent document with publication number US20050125037A1 refers to embodiments in which the suture thread is shaped by heat plates. In this embodiment, there are the said multiple production problems.

In the USPTO patent document with publication number US5643628A discloses the method of suture thread production, which is automatically applied with adhesive.

The application with the number US5156788A pertains to surgical sutures, specifically a method for treating suture ends to facilitate their insertion into surgical needles. Traditional methods involve using resin solutions or melt fusion to stiffen suture ends, but these techniques present issues such as inconsistency, loss of tensile strength, and permanent stiffness. The disclosed invention introduces a heat tipping method that provides a reversible stiffening effect without compromising the suture's tensile strength. The process involves heating a delimited portion of the suture to a specific temperature (300-320°F) using a controlled airflow system and then allowing it to cool, either passively or actively. This method ensures uniform and consistent results, prevents filament fusion, and maintains the flexibility of the suture after insertion into a needle. The invention also includes an apparatus featuring a cylindrical drum with a groove to delimit the heating area, a hot air outlet for heating, and a cooling air outlet to protect the rest of the suture. This process effectively eliminates fraying while ensuring ease of use and reversibility of stiffness.

The US5250247A relates to surgical sutures, specifically to a method for treating suture ends to facilitate their insertion into surgical needles. Traditional methods involve coating or melting the suture tip, but these approaches can cause inconsistencies, stiffness, and loss of tensile strength. The disclosed method introduces a heat tipping process that provides reversible stiffening without degrading the suture. By selectively heating a portion of the suture to a controlled temperature (300-320°F) and allowing it to cool, the method ensures even and consistent results while preventing fraying. The invention also includes an apparatus featuring a cylindrical drum with a groove for precise heating and cooling air outlets to protect non-heated areas. This process improves handling and usability without permanently altering the suture's flexibility.

US5672375A discloses an automated method and apparatus for tipping, cutting, and sorting surgical sutures. The system streamlines suture preparation by feeding sutures from a reel, applying a tipping agent-preferably cyanoacrylate-to stiffen the suture tip, optionally drying the tipped portion, measuring the suture diameter, and then precisely cutting and sorting sutures based on predefined criteria. The apparatus utilizes computer-controlled gripping arms to position the suture through each stage, ensuring consistent tipping, reducing filament fraying, and maintaining suture integrity. By automating these processes, the invention enhances efficiency, accuracy, and quality in the production of surgical sutures, facilitating their use in needle-suture combinations.

The US5569302A relates to a tipped multifilament surgical suture and a method for its production. Specifically, it introduces the use of a cyanoacrylate tipping agent to prevent "brooming" (fraying of the suture end) and increase stiffness, thereby facilitating suture attachment to a surgical needle. The document reviews various prior methods of securing sutures to needles, such as using adhesives, swaging, or tubing attachments, highlighting their limitations. The disclosed invention employs a controlled process where sutures are wound onto a drum, tension-adjusted for consistent diameter, and then exposed to an atomized mist of cyanoacrylate, which quickly polymerizes upon contact with ambient moisture. This results in a stiffened tip suitable for insertion into a needle bore. The method ensures compatibility with various suture materials, including coated or filled sutures, and provides superior pull-out strength compared to conventional tipping techniques. The invention further includes an apparatus for precise and repeatable tipping, incorporating automated tension control, mist application, and nitrogen flushing to maintain process consistency.

The French application FR2526453A1 relates to a cord, a method for treating the cord, and an installation for implementing this method, with particular application to laces, cords, and straps, including the treatment of their tips. Traditional methods of preventing fraying, such as knots, metal clips, or cellophane coatings, have various drawbacks, including aesthetic concerns, oxidation risks, fragility, and the need for precise sizing. For synthetic and textile cords, cutting with a hot blade can lead to uncontrolled melting, deformation, and discoloration. The invention aims to provide a cord with a treated portion that remains rigid, facilitating easy insertion into shoe eyelets while preventing fraying, without compromising color or durability. The treatment method involves tightening the cord's fibers, fusing them together through controlled heating, and optionally using varnish for natural fibers. The invention also includes an industrial installation capable of processing multiple cords simultaneously, ensuring high production efficiency at a low cost. The described system employs tensioning mechanisms, hot air treatment, and controlled cutting to create durable, aesthetically pleasing cord tips suitable for mass production.

The publication with number US8222564B2 relates to a method for modifying a surgical fiber to facilitate its attachment to a surgical needle. Traditional mechanical machining methods, such as cutting, grinding, and milling, are slow and imprecise due to tool wear. This invention introduces a process that utilizes an irradiating device, such as a laser, to selectively remove material from the surgical fiber, creating a reduced portion that enables secure coupling with a surgical needle. The laser beam can be directed transversely or along the fiber's axis, and a curved reflector may be used to achieve uniform irradiation from multiple angles. The surgical fiber may be held in a rotating or translating holder to ensure precise material removal. The method allows for controlled shaping of the reduced portion, including non-uniform topographies like threads, ribs, or barbs to enhance anchoring within the needle's receiving structure. Additionally, an alternative technique removes material from an internal region of the fiber, forming a cavity that can collapse under applied force to reduce the outer dimension. Another variation employs multiple irradiating devices for efficient, even modification of the fiber. The process improves precision, consistency, and production efficiency compared to traditional methods, making it suitable for large-scale manufacturing of surgical sutures.

All the problems mentioned above have made it necessary to make an innovation in the relevant field as a result.

### THE OBJECTS OF THE INVENTION

The present invention is put forward to eliminate the aforementioned problems and to make a technical innovation in the relevant field.

The main object of the invention is to reveal the machine structure that enables the threads to be cut and the ends to be hardened in order to ensure that a large number of suture threads are prepared automatically in the unit time.

Another object of the invention is to prevent the increase of ambient temperature by operating the heat source only during the hardening process.

Another object of the invention is to ensure that the heat is collected only in the area to be hardened with special reflectors of the heat source.

Another object of the invention is to prevent the dispersion of infrared rays with the reflectors of the heat source.

Another object of the invention is to ensure that the wastes in production are kept to a minimum by cutting the thread at the specified angle.

Another object of the invention is to allow high capacity mass production with the machine located in a small area.

Another object of the invention is to allow production to a certain standard by ensuring that the hardened parts of all cut and hardened threads are equal.

Another object of the invention is to prevent the deterioration/loss of quality of the threads by heating the environment during the hardening process.

### BRIEF DESCRIPTION OF THE INVENTION

The present invention is defined by the features of the independent claims. Embodiments of the invention are defined in the dependent claims.

The present invention is a surgical thread cutting and hardening machine in order to realize all the objects that will emerge from the above mentioned and the following detailed description.

Another preferred embodiment of the disclosure comprises at least two winding members connected to the side plate on both sides, forming the outer edges to enable the threads to be wound on the drum rotatable on its axis.

Another preferred embodiment of the disclosure comprises a drum having at least one lower reflector positioned inwards from the outer edge of the said successive winding members between two successive winding members.

Another preferred embodiment of the disclosure comprises more than two winding members fixed between two side plates in such a way that the linear length in a turn around the axis of the period is the desired length of the thread when the thread is wound on the drum in accordance with the length of the thread to be cut.

Another preferred embodiment of the disclosure comprises a thread transfer mechanism with a tension setting mechanism that allows the tension of the thread to be wound on it by rotating the drum with mechanical force on its axis.

Another preferred embodiment of the disclosure comprises a thread transfer slide positioned parallel to the drum and a thread transfer carriage that can be moved linearly on the thread transfer slide in order to ensure that the pulley with the thread to be wound on the drum is aligned with the drum and the thread on the drum is oriented not to overlap.

Another preferred embodiment of the disclosure comprises a drum fixed to the side plate comprising a lower reflector fixing opening positioned in the same direction with each other to enable the lower reflector to be fixed.

Another preferred embodiment of the disclosure comprises a drum axis member located at the center of at least one of the side plates to allow the drum to rotate on a single axis.

Another preferred embodiment of the disclosure comprises at least two lower reflector bodies to ensure that the lower reflector is fixed on the side plate.

Another preferred embodiment of the disclosure comprises at least one lower reflector retaining bracket to ensure that the lower reflector is fixed on the lower reflector body.

Another preferred embodiment of the disclosure comprises at least two drum brackets that allow the drum to be fixed on the machine body and to be bedded over the drum axis members.

Another preferred embodiment of the disclosure comprises a cutter that enables the threads to be cut with the knife that moves linearly on at least one slide so as to move parallel to the drum positioned between the heater/cutter side bodies in order to ensure the cutting of the threads wound on the drum along the drum.

Another preferred embodiment of the disclosure comprises a circular knife rotated by at least one electric motor on its axis to be in contact at an angle of 45° with the threads.

Another preferred embodiment of the disclosure comprises a heater which is an IR heater.

Another preferred embodiment of the disclosure comprises at least one heater bracket that enables the heater to be fixed in the upper reflector.

Another preferred embodiment of the disclosure comprises an upper reflector with an outer radius to provide a homogeneous reflection of the IR rays.

Another preferred embodiment of the disclosure has a lower reflector comprising an inner radius, which allows the threads to be rigidly hardened by reversing the rays reflected from the upper reflector or forming a distance to prevent the contact of the cutter with the lower reflector during cutting.

Another preferred embodiment of the disclosure comprises a lower reflector and upper reflector that are made of metal material and allow the IR light to be reflected.

Another preferred embodiment of the disclosure comprises two side panels positioned at the edges of the upper reflector to hold the heater completely inside and prevent the reflectance of IR light outside when it joins the lower reflector.

Another preferred embodiment of the disclosure has which has the structure of the heating unit and the cutter mechanism positioned in different positions on the heater/cutter mechanism in order to determine the working position by rotating on the same axis, and which has the process of rotating the heater/cutter mechanism on its axis and approximating the heater/cutter mechanism to the drum with a linear movement to ensure the approximation of the relevant unit to the drum according to the operation to be performed on the thread wound on the drum. The scope of protection of the invention is specified in the claims and cannot be limited to those explained for sampling purposes in this brief and detailed description. It is evident that a person skilled in the art may exhibit similar embodiments in light of the above-mentioned facts without drifting apart from the main theme of the invention.

### BRIEF DESCRIPTION OF THE DRAWINGS

Figure 1 shows a front view of the machine.
Figure 2 shows a perspective view of the cutting machine.
Figure 3 shows a perspective view of the drum with the brackets.

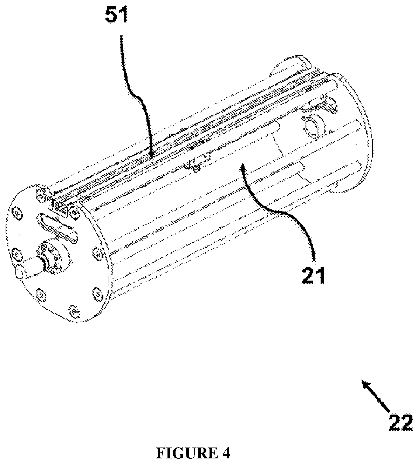

Figure 4 shows a perspective view of the drum.
Figure 5 shows a perspective cold of the drum and the lower reflector part on the drum.
Figure 6 shows a perspective view showing the positioning of the upper reflector and the lower reflector with the source.
Figure 7 shows a cross-sectional view of the reflector and source.
Figure 8 shows a perspective view of the reflector and source.
Figure 9 shows a perspective view of the mechanism that enables the transfer of the thread of the invention.
Figure 10 shows the drawing showing the cutting angle of the cutter.

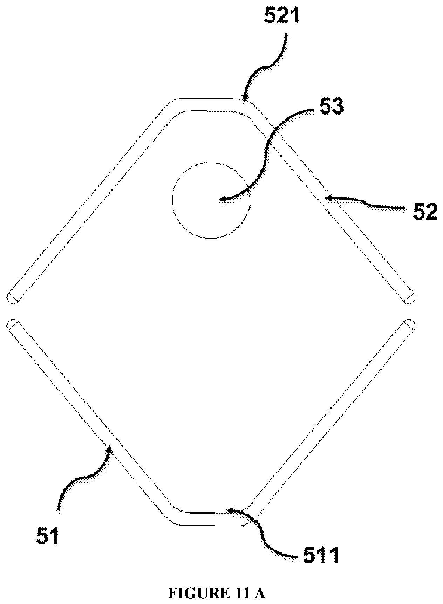

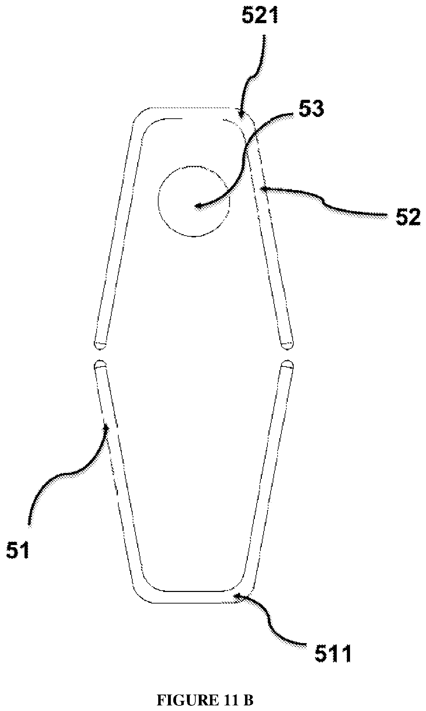

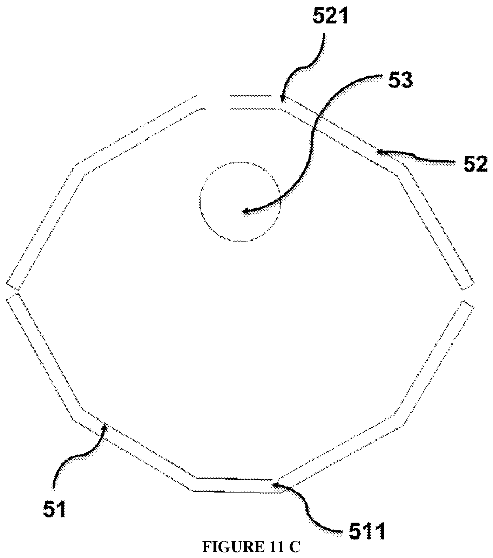

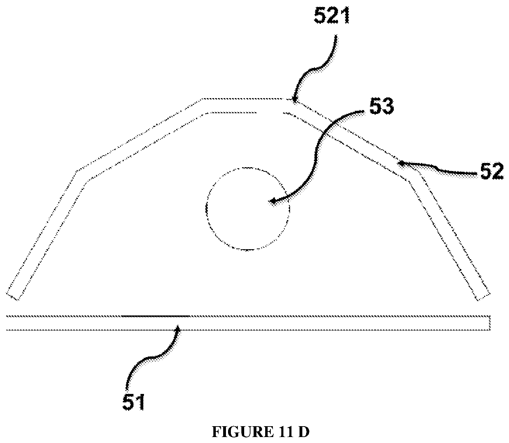

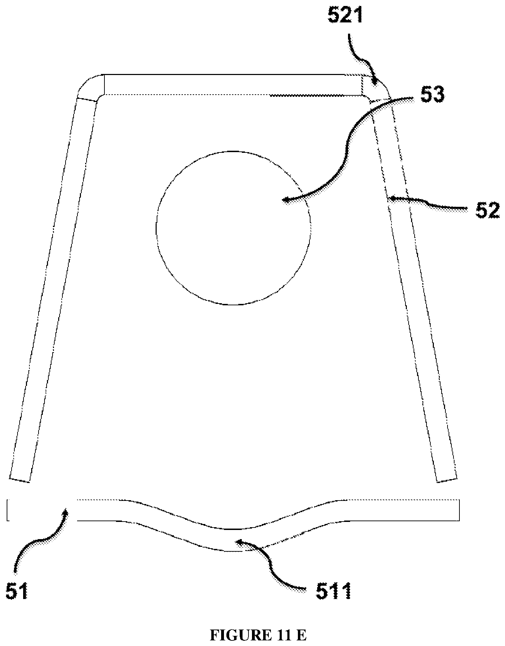

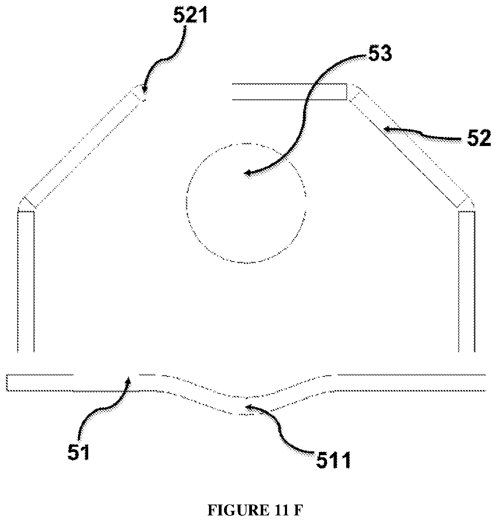

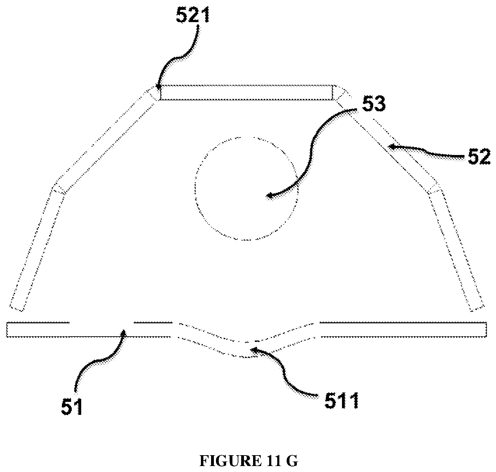

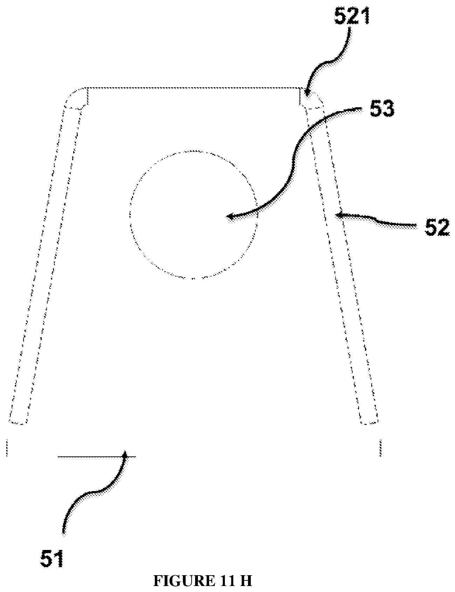

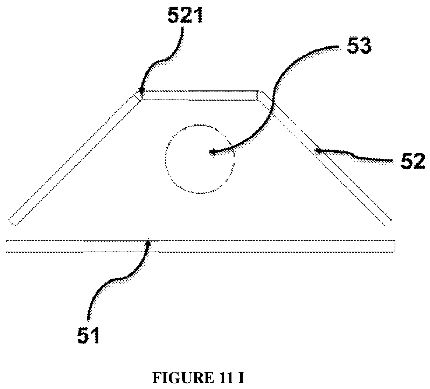

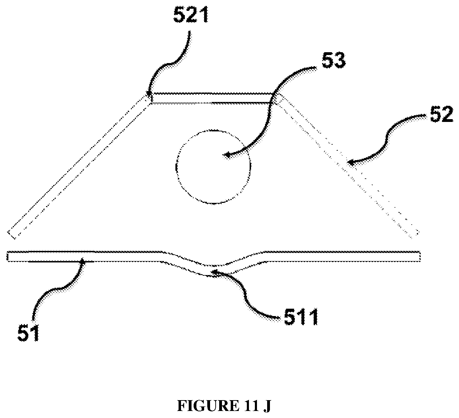

Figures 11 A, B, C, D, E, F, G, I, J shows alternative embodiments of the reflector section.

### DESCRIPTION OF THE REFERENCE NUMBERS IN THE FIGURES

10. Machine body
   11. Controller
20. Winding mechanism
   21. Drum
      210. Side plate
         2101. Lower reflector fixing opening
         2102. Centering flange
         2103. Drum axis member
      211. Winding member
         2110. Winding member fixing bolt
   22. Drum bracket
30. Heater/cutter mechanism
   300. Heater/cutter mechanism side body
   31. Cutter
   311. Knife
40. Thread transfer mechanism
   41. Thread transfer slide
   42. Thread transfer carriage
   43. Pulley
   44. Tension setting mechanism
50. Heating unit
   51. Lower reflector
      510. Lower reflector body
      511. Inner radius
      512. Lower reflector retaining bracket
   52. Upper reflector
      521. Outer radius
   53. Heater
      531. Heater bracket
   M. Machine

### DETAILED DESCRIPTION OF THE INVENTION

In this detailed description, the **cutting and hardening machine for surgical threads** of the invention is explained only with examples that will not have any limiting effect in order to better understand the subject matter.

The disclosure relates to a machine (M) that enables multiple cutting of surgical multifilament threads and hardening of the ends to connect them to the needle after cutting.

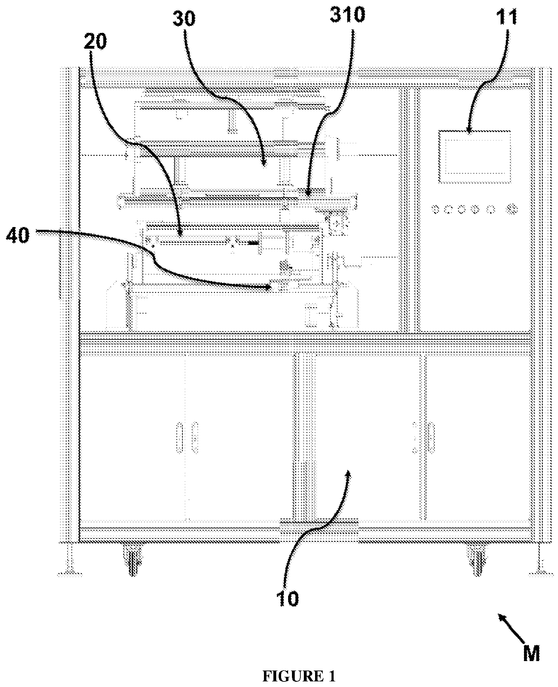

Figure 1 shows a front view of the disclosure. The machine body (10) includes a wrapping mechanism (20) and a heater/cutter mechanism (30), which are at least two independently movable parts. The cutter (31), which has a knife (311) that allows the threads to be cut by intervening on the wrapping mechanism (20), has at least one thread transfer mechanism (40) to enable the threads to be wrapped on the wrapping mechanism (20). The thread transfer mechanism (40) allows the thread to be wrapped rigidly at the specified tension when winding on the drum (21) that the thread wrapping mechanism (20) has.

The heater/cutter mechanism (30) of the invention preferably houses the cutter mechanism (310) and the heating unit (50) positioned 180° with each other. The correct control and supervision of the moving mechanisms are carried out by the controller (11).

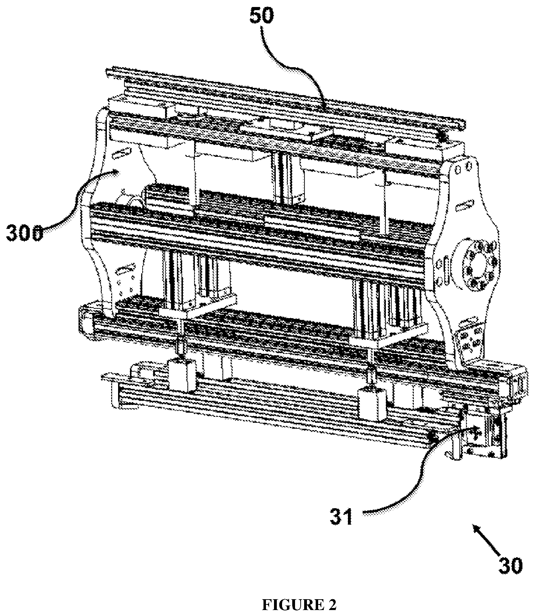

Figure 2 shows a perspective view of the heater/cutter mechanism (30) of the disclosure. In the said embodiment, the heating unit (50) and the cutter mechanism (310) are gathered in the same unit together with the heater/cutter mechanism side body (300), and it is practically ensured that the selected unit is activated according to the preferred process moment. The working units are preferably the units remaining at the lower part and are working on the winding mechanism (20). In alternative embodiments of the disclosure, these positions may vary or the wrapping mechanism (20) may be positioned on the lower and upper parts of the machine (M). By rotating the heater/cutter mechanism (30) of the disclosure in its axis, the position of the cutter mechanism (31) or the heating unit (50) can be controlled. In the preferred embodiment of the disclosure, the heater/cutter mechanism (30) can be rotated by at least one electric motor from the axis of the heater/cutter mechanism side body (300). The correct position can be monitored instantly by the controller (11) with the position sensors used. The heater/cutter mechanism (30) can rotate on its axis and move linearly on the vertical axis.

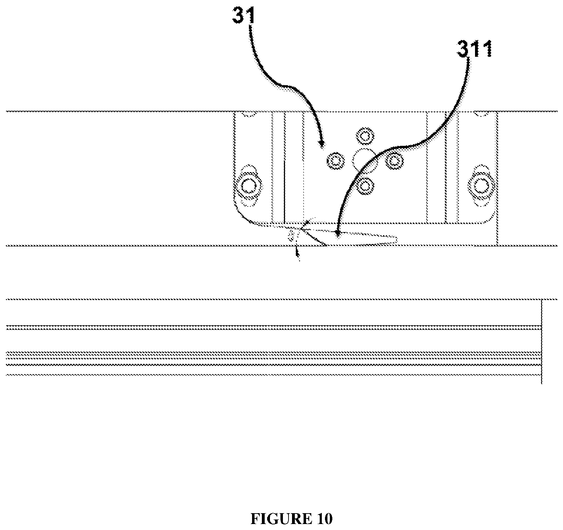

Figure 10 shows the side view of the cutter (31) of the disclosure. The cutter (31) can move linearly in the slides on the heater/cutter mechanism (30). The said movement is preferably controlled by at least one servo motor and can also be controlled manually in alternative embodiments. The linear movement preferably takes place perpendicular to the thread direction wound on the drum (21). In the preferred embodiment, at the point where the knife (311) of the cutter (31) contacts the thread, there is a 45° angle between the thread and the knife (311). In this way, the thread is cut in the most effective way. The said angle can be seen in Figure 10.

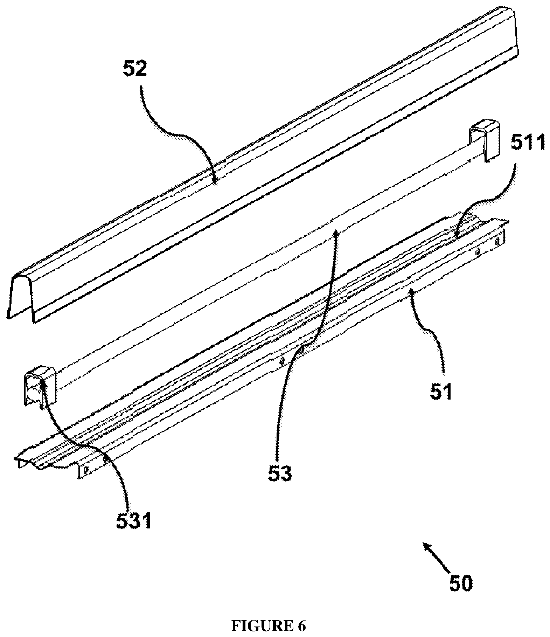

Figures 6, 7, and 8 show detailed drawings of the heating unit (50) located on the heater/cutter mechanism (30). The said heating unit (50) is positioned with the upper reflector (52) at the top and the lower reflector (51) at the bottom during the application. The heater (53) is positioned between the lower reflector (51) and the upper reflector (52) and is connected to the upper reflector (52). The connection is preferably made by the heater bracket (531). The movement of the upper reflector (52) and the heater (53) is controlled by the heater/cutter mechanism (30). The control of the movement of the upper reflector (52) and the members connected to the upper reflector (52) is controlled by the movement of the heater/cutter mechanism (30).

The lower reflector (51) of the heating unit (50) is preferably fixed on the drum (21) and is movable only by the movement of the drum (21).

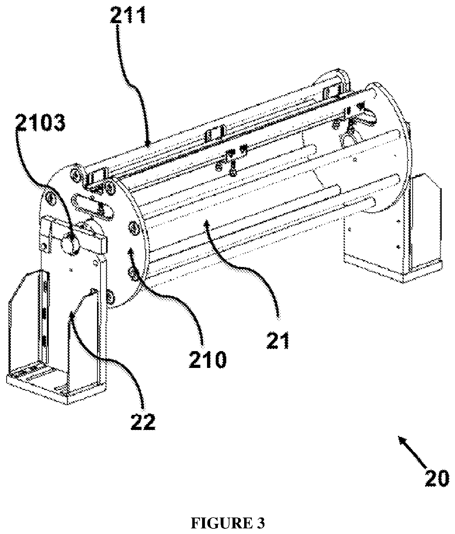

Figure 3 shows the perspective cold of the winding mechanism (20) of the disclosure. The said wrapping mechanism (20) includes a drum (21) that allows the thread to be wrapped and processed. The said drum (21) is connected to the machine body (10) by at least two drum brackets (22). By rotating the drum (21) on its axis, the thread is wound on it. Furthermore, cutting and hardening of the thread by the units on the heater/cutter mechanism (30) takes place on the drum (21).

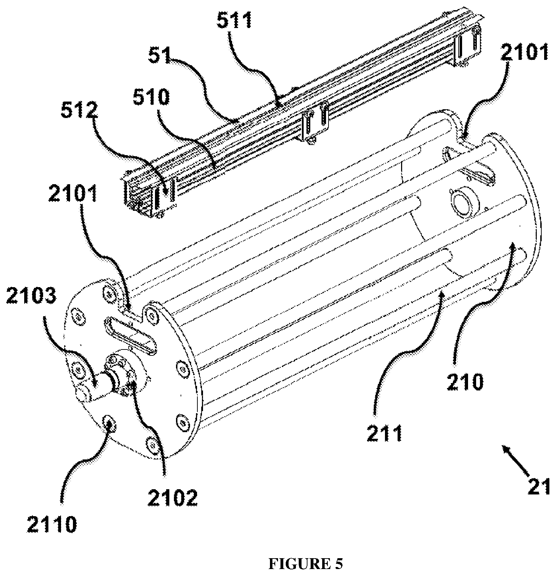

The said drum (21) comprises at least three winding members (211) positioned between at least two side plates (210). The winding members (211) are fixed on the side plates (210) by the winding member fixing bolts (2110) and the thread is wound on the winding members (211) by rotating the drum (21) in the axis of the side plates (210). The said drum (21) is supported to the drum bracket (22) by at least one drum axis member (2103) located in the center of the side plates (210). A detailed drawing of the drum (21) is given in Figure 5. The drum axis members (2103) are adapted to the center of the side plate (210) by the mating flanges (2102). The rotation of the drum (21) is preferably carried out by at least one electric motor.

In the preferred embodiment of the disclosure, the winding members (211) fixed between the two side plates (210) to form the drum (21) are shafts with cylindrical surface form.

There is at least one lower reflector fixing opening (2101) on the two side plates (210) forming the drum (21). The lower reflector fixing opening (2101) is positioned between two successive winding members (211), on which the lower reflector (51) is fixed. By means of the said opening, the lower reflector (51) is provided to be lower than the peak of the two winding members (211) following the lower reflector (51) away from the center. In this way, during the winding of the thread to the winding members (211) on the drum (21), the lower reflector (51) is located at the bottom of the winding threads, in the inner part of the drum (21).

As shown in Figure 5, there are alternative embodiments that enable the said lower reflector (51) to be fixed on the drum (21). In the preferred embodiment, the lower reflector (51) can be fixed by the lower reflector retaining brackets (512) located on the lower reflector body (510). Preferably, the lower reflector body (510) enables the lower reflector (51) to be connected to the drum (21).

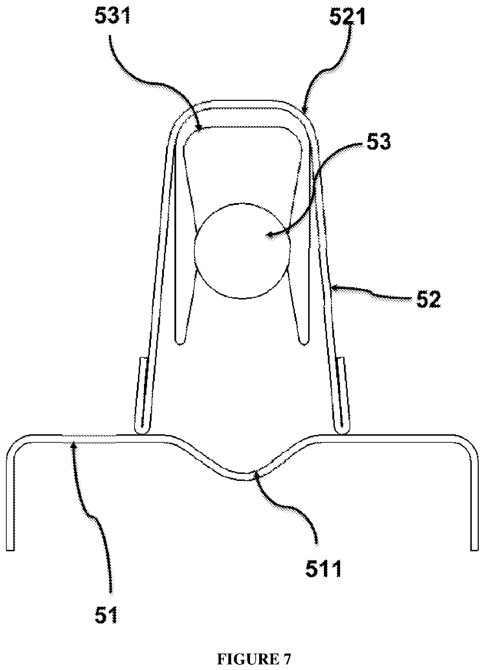

In the preferred embodiment of the disclosure, there is at least one radius (51) on the lower reflector (51). It provides an optimum reflection of the rays by the inner radius (51). Figure 7 shows a cross-sectional view of the heating unit (50). When the upper reflector (52) on the heater/cutter mechanism (30) is positioned on the drum (21), it is positioned on the lower reflector (51). The heater (53) is located inside the upper reflector (52). In the preferred embodiment, the upper reflector (52) of the disclosure is positioned on the outer radius (521) at the upper part. In the preferred embodiment, the heater (53) is fixed to the upper reflector (52) by means of the heater bracket (531). The threads wound on the drum (21) and to be hardened are located between the upper reflector (52) and the lower reflector (51). The part of the threads that are wound on the threads by the rays transmitted by the heater (53) is provided to harden. It is ensured that the form of the upper reflector (52) and the internal radius (511) of the lower reflector (51) reflect the IR rays coming out of the heater (53) in the interior of the reflectors in an optimum way and that the hardening process is best performed.

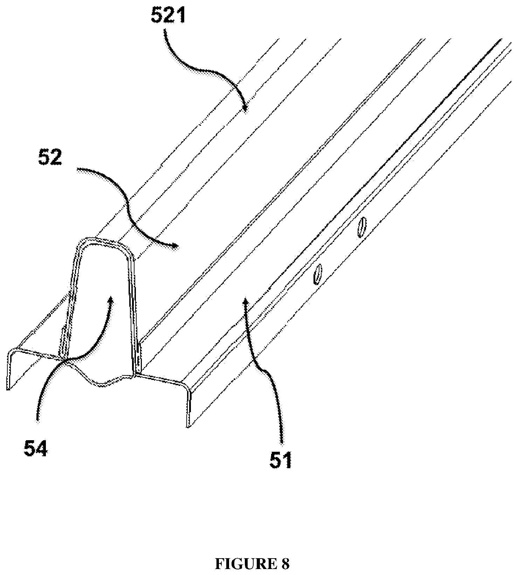

Figure 8 shows the position of the reflectors during operation. When the upper reflector (52) and the lower reflector (51) are closed by means of the side panel on both sides connected or monolithic on the upper reflector (52), the leakage of IR light from the inside to the outside is prevented.

In the preferred embodiment of the disclosure, the upper reflector (52) comprises an antireflection plate enabling the edges thereof to be closed. In this way, the leakage/passage of rays into the environment is prevented while the hardening process is carried out.

The heater (53) used in the preferred embodiment of the disclosure is preferably an IR heater.

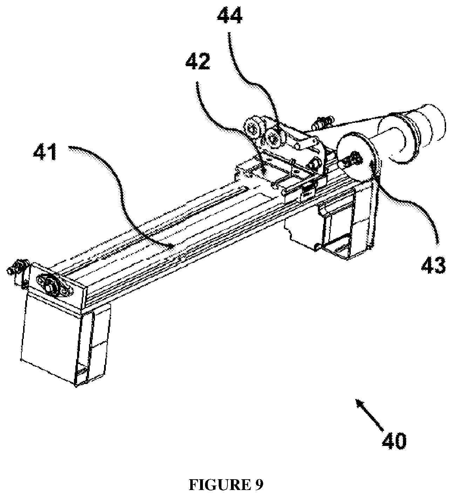

Figure 9 shows a perspective view of the thread transfer mechanism (40). The thread transfer mechanism (40) controls the winding of the thread on the coil on the drum (21) in the desired order and tension. The thread transfer mechanism (40) is positioned parallel to the drum (21) and can move linearly in this direction. The thread transfer slide (41) is positioned parallel to the drum (21) and the thread transfer carriage (42) on the thread transfer slide (41) can move linearly. The said linear movement can be controlled by at least one stepping motor or servo motor. The tension of the thread taken from the pulley (43) is adjusted by the tension setting mechanism (44) on the thread transfer carriage (42). The winding process on the drum (21) is carried out by rotating the drum (21), and the thread transfer carriage (42) moves linearly in order not to overlap the wound threads.

After a sufficient amount of thread is wound on the drum (21), the rotation of the drum (21) stops. The drum (21) is positioned to remain in the top position of the lower reflector (51) unit on it. During the hardening process, it is ensured that the upper reflector (52) unit, which is housed by the heating unit (50) of the heater/cutter mechanism (30) that can rotate in its axis, is positioned on the lower reflector (51) on the drum (21). The said action is realized by the linear movement of the heater/cutter mechanism (30) in the direction of the drum. By energizing the heater (53) for the specified time, an IR ray is emitted on the threads wound on the drum (21) and the threads are hardened.

After the threads have hardened, the heater/cutter mechanism (30) rises linearly and moves linearly in the direction of the drum (21) by positioning the cutter mechanism (300) in the direction of the drum (21). The knife (311) of the cutter (31) is a circular release and is rotated by at least one electric motor. The process is completed by cutting the threads from the part where they harden.

The system of the invention operates completely automatically and movement is provided by electromechanical or pneumatic systems controlled by the controller (11).

In the preferred embodiment of the disclosure, the movement of the heater/cutter mechanism (30) is controlled pneumatically.

In the preferred embodiment of the disclosure, the lower reflector (51) and the upper reflector (52) are made of material that will reflect the IR rays. In an alternative embodiment, the interior may comprise a coating.

Within the scope of the disclosure, the synchronous operation can be carried out by using more than one drum (21) on the same machine, and the production capacity can be increased by extending the length of the machine (M). In addition, regional heating is performed in the hardening process with the IR heater and the heating of the environment is prevented.

## Claims

1. A machine (M), which provides hardening and cutting of surgical threads and prepares the thread end to be placed on the suture needle to prevent fraying, comprises a heater/cutter mechanism (30) containing a cutter mechanism (310) and a heating unit (50), a winding mechanism (20) which is aligned with the said heater/cutter mechanism (30) on at least one axis, a drum (21) which enables a thread to be wound thereon by rotating on its axis on the said winding mechanism (20), a lower reflector (51) which consists of at least one part on the said drum (21), and at least one cutter (31) on a cutter mechanism (310) to enable the threads wound on the drum (21) to be cut, **characterized in that** the cutter mechanism (310) is rotatable and linearly movable on its axis and the heating unit (50) is rotatable with the rotatable heater/cutting mechanism (30), at least one upper reflector (52) on the heating unit (50) which provides heating of the threads wound on the drum (21) when the said upper reflector (52) is aligned with the lower reflector (51), wherein at least one heater (53) is located inside of the upper reflector (52).

2. The machine (M) according to claim 1, wherein it comprises at least two winding members (211) connected to a side plate (210) on both sides, forming outer edges to enable the threads to be wound on the drum (21) that can rotate in its axis.

3. The machine (M) according to claim 1, wherein the lower reflector (51) is positioned between two successive winding members (211) of said drum (21) such that it is located more inwardly than the outer edge of the said successive winding members (211),
it further comprises more than two winding members (211) fixed between the two side plates (210) so that, when the thread is wound on the drum (21) in accordance with the length of the thread to be cut, the linear length in a round around the rotation axis is the desired thread length.

4. The machine (M) according to claim 1, comprising a thread transfer mechanism (40) with a tension setting mechanism (44) that allows the tension of the thread to be wound by rotation of the drum (21) on its axis with mechanical force.

5. The machine (M) according to claim 1, comprising a thread transfer slide (41) positioned parallel to the drum (21) to ensure that a pulley (43) including the thread to be wound on the drum (21) is aligned to the drum (21) and that the thread on the drum (21) is oriented not to overlap, and a thread transfer carriage (42) that can move linearly on the said thread transfer slide (41).

6. The machine (M) according to claim 1, comprising a drum (21) fixed to the side plate (210) comprising a lower reflector fixing opening (2101) positioned in the same direction with each other to enable the lower reflector (51) to be fixed.

7. The machine (M) according to claim 1, comprising a drum axis member (2103) positioned at the center of at least one of the side plates (210) to allow the drum (21) to rotate in a single axis.

8. The machine (M) according to claim 1, comprising at least two lower reflector bodies (510) to enable the lower reflector (51) to be fixed on the side plate (210).

9. The machine (M) according to claim 1, comprising at least one lower reflector retaining bracket (512) to allow the lower reflector (51) to be fixed on the lower reflector body (510).

10. The machine (M) according to claim 1, comprising at least two drum brackets (22) that enable the drum (21) to be fixed on the machine body (10) and to be housed over the drum axis members (2103).

11. The machine (M) according to claim 1, comprising the cutter (31) that enables the threads to be cut with a knife (311) that moves linearly on at least one slide so as to move parallel to the drum (21) positioned between a heater/cutter mechanism side body (300) in order to ensure the cutting of the threads wound on the drum (21) along the drum (21).

12. The machine (M) according to claim 1, comprising at least one heater bracket (531), which enables the heater (53) to be fixed in the upper reflector (52).

13. The machine (M) according to claim 1, comprising the lower reflector (51) with an inner radius (511) which provides rigid hardening of the threads by inverse reflecting of the rays reflected from the upper reflector (52) or forming a distance to prevent the contact of the cutter (31) with the lower reflector (51) during cutting.

14. A method of preventing fraying by hardening the ends of the threads to be cut at the desired dimensions by wrapping the surgical threads on at least one cylindrical form of a machine (M) according to any previous claim 1-13; wherein said thread is kept between the lower reflector (51) and the upper reflector (52) by covering the threads wound on the drum (21); wherein the lower reflector (51) is configured to be aligned exactly with the upper reflector (52) of the heating unit (50) by rotating the drum (21) on its axis; wherein a regional heating process is carried out in the threads by irradiation in the closed area created by the upper reflector (52) and the lower reflector (51) with at least one infrared heater in the said upper reflector (52).

15. The method according to claim 14, which has the structure of the heating unit (50) and the cutter mechanism (310) positioned in different positions on the heater/cutter mechanism (30) in order to determine the working position by rotating on the same axis, and which has the processes of rotating the heater/cutter mechanism (30) on its axis and approximating the heater/cutter mechanism (30) to the drum (21) with a linear movement to ensure the approximation of the relevant unit to the drum (21) according to the operation to be performed on the thread wound on the drum (21).

## Patentansprüche

1. Maschine (M), die chirurgische Fäden härtet und schneidet und das Fadenende für die Aufnahme in die Nahtnadel vorbereitet, um ein Ausfransen zu verhindern, umfasst einen Heiz-/Schneidemechanismus (30) mit einem Schneidemechanismus (310) und einer Heizeinheit (50), einen Wickelmechanismus (20), der mit dem Heiz-/Schneidemechanismus (30) auf mindestens einer Achse ausgerichtet ist, eine Trommel (21), auf die ein Faden durch Drehen um ihre Achse auf dem Wickelmechanismus (20) aufgewickelt werden kann, einen unteren Reflektor (51), der aus mindestens einem Teil auf der Trommel (21) besteht, und mindestens einen Schneider (31) an einem Schneidemechanismus (310), um das Schneiden der auf die Trommel (21) aufgewickelten Fäden zu ermöglichen, **dadurch gekennzeichnet, dass** der Schneidemechanismus (310) um seine Achse drehbar und linear beweglich ist und die Heizeinheit (50) mit dem drehbaren Heiz-/Schneidemechanismus (30) drehbar ist, mindestens ein oberer Reflektor (52) an der Heizeinheit (50), der die auf die Trommel (21) gewickelten Fäden erwärmt, wenn der obere Reflektor (52) mit dem unteren Reflektor (51) ausgerichtet ist, wobei sich mindestens eine Heizvorrichtung (53) innerhalb des oberen Reflektors (52) befindet.

2. Maschine (M) gemäß Anspruch 1, wobei sie mindestens zwei Wickelelemente (211) umfasst, die an beiden Seiten mit einer Seitenplatte (210) verbunden sind und Außenkanten bilden, damit die Fäden auf die Trommel (21) gewickelt werden können, die sich um ihre Achse drehen kann.

3. Maschine (M) gemäß Anspruch 1, wobei der untere Reflektor (51) zwischen zwei aufeinanderfolgenden Wickelelementen (211) der Trommel (21) so positioniert ist, dass er weiter innen liegt als die Außenkante der aufeinanderfolgenden Wickelelemente (211),
sie umfasst ferner mehr als zwei Wickelelemente (211), die zwischen den beiden Seitenplatten (210) befestigt sind, so dass, wenn der Faden entsprechend der Länge des zu schneidenden Fadens auf die Trommel (21) gewickelt wird, die lineare Länge in einer Runde um die Drehachse die gewünschte Fadenlänge ist.

4. Maschine (M) gemäß Anspruch 1, umfassend einen Fadenübertragungsmechanismus (40) mit einem Spannungseinstellmechanismus (44), der es ermöglicht, die Spannung des Fadens durch Drehen der Trommel (21) um ihre Achse mit mechanischer Kraft einzustellen.

5. Maschine (M) gemäß Anspruch 1, umfassend einen Fadenübertragungsschlitten (41), der parallel zur Trommel (21) positioniert ist, um sicherzustellen, dass eine Rolle (43) mit dem auf die Trommel (21) aufzuwickelnden Faden auf die Trommel (21) ausgerichtet ist und dass der Faden auf der Trommel (21) so ausgerichtet ist, dass er sich nicht überlappt, und einen Fadenübertragungswagen (42), der sich linear auf dem Fadenübertragungsschlitten (41) bewegen kann.

6. Maschine (M) gemäß Anspruch 1, umfassend eine Trommel (21), die an der Seitenplatte (210) befestigt ist und eine untere Reflektorbefestigungsöffnung (2101) umfasst, die in derselben Richtung zueinander positioniert ist, um die Befestigung des unteren Reflektors (51) zu ermöglichen.

7. Maschine (M) gemäß Anspruch 1, umfassend ein Trommelachsenelement (2103), das in der Mitte mindestens einer der Seitenplatten (210) positioniert ist, damit sich die Trommel (21) um eine einzige Achse drehen kann.

8. Maschine (M) gemäß Anspruch 1, umfassend mindestens zwei untere Reflektorkörper (510), um die Befestigung des unteren Reflektors (51) an der Seitenplatte (210) zu ermöglichen.

9. Maschine (M) gemäß Anspruch 1, umfassend mindestens eine untere Reflektorbefestigungshalterung (512), um den unteren Reflektor (51) am unteren Reflektorkörper (510) zu befestigen.

10. Maschine (M) gemäß Anspruch 1, umfassend mindestens zwei Trommelhalterungen (22), die es ermöglichen, die Trommel (21) am Maschinenkörper (10) zu befestigen und über den Trommelachsenelementen (2103) unterzubringen.

11. Maschine (M) gemäß Anspruch 1, umfassend den Schneider (31), der das Schneiden der Fäden mit einem Messer (311) ermöglicht, das sich linear auf mindestens einem Schlitten bewegt, um sich parallel zu der Trommel (21) zu bewegen, die zwischen einem Heiz-/Schneidemechanismus-Seitenkörper (300) positioniert ist, um das Schneiden der auf die Trommel (21) gewickelten Fäden entlang der Trommel (21) sicherzustellen gewährleistet wird.

12. Maschine (M) gemäß Anspruch 1, umfassend mindestens eine Heizhalterung (531), die es ermöglicht, die Heizvorrichtung (53) im oberen Reflektor (52) zu befestigen.

13. Maschine (M) gemäß Anspruch 1, umfassend den unteren Reflektor (51) mit einem Innenradius (511), der eine starre Verfestigung der Fäden durch umgekehrte Reflexion der vom oberen Reflektor (52) reflektierten Strahlen oder durch Bildung eines Abstands bewirkt, um den Kontakt des Schneiders (31) mit dem unteren Reflektor (51) während des Schneidens zu verhindern.

14. Verfahren zum Verhindern von Ausfransen durch Härten der Enden der Fäden, die auf die gewünschten Abmessungen zugeschnitten werden sollen, indem die chirurgischen Fäden auf mindestens eine zylindrische Form einer Maschine (M) gemäß einem der vorherigen Ansprüche 1-13 gewickelt werden, wobei der Faden zwischen dem unteren Reflektor (51) und dem oberen Reflektor (52) gehalten wird, indem die auf die Trommel (21) gewickelten Fäden abgedeckt werden, wobei der untere Reflektor (51) so konfiguriert ist, dass er durch Drehen der Trommel (21) um ihre Achse genau mit dem oberen Reflektor (52) der Heizeinheit (50) ausgerichtet wird, wobei ein regionaler Erhitzungsprozess in den Fäden durch Bestrahlung in dem durch den oberen Reflektor (52) und den unteren Reflektor (51) geschaffenen geschlossenen Bereich mit mindestens einem Infrarotheizer in dem oberen Reflektor (52) erzeugt wird.

15. Verfahren nach Anspruch 14, bei dem die Heizeinheit (50) und der Schneidemechanismus (310) an unterschiedlichen Positionen am Heiz-/Schneidemechanismus (30) angeordnet sind, um die Arbeitsposition durch Drehen um dieselbe Achse zu bestimmen, und die Verfahren umfasst, bei denen der Heiz-/Schneidemechanismus (30) um seine Achse gedreht und der Heiz-/Schneidemechanismus (30) mit einer linearen Bewegung an die Trommel (21) angenähert wird, um die Annäherung der entsprechenden Einheit an die Trommel (21) entsprechend dem an dem auf der Trommel (21) aufgewickelten Faden auszuführenden Vorgang sicherzustellen.

## Revendications

1. Une machine (M) qui assure le durcissement et la coupe de fils chirurgicaux et prépare l'extrémité du fil à placer sur l'aiguille à suture afin d'empêcher l'effilochage, comprend un mécanisme de chauffage/coupe (30) contenant un mécanisme de coupe (310) et une unité de chauffage (50), un mécanisme d'enroulement (20) qui est aligné avec ledit mécanisme de chauffage/coupe (30) sur au moins un axe, un tambour (21) qui permet d'enrouler un fil en tournant sur son axe sur ledit mécanisme d'enroulement (20), un réflecteur inférieur (51) qui se compose d'au moins une partie sur ledit tambour (21), et au moins une coupeuse (31) sur un mécanisme de coupe (310) pour permettre de couper les fils enroulés sur le tambour (21), **caractérisé en ce que** le mécanisme de coupe (310) est rotatif et mobile linéairement sur son axe et que l'unité de chauffage (50) est rotative avec le mécanisme rotatif de chauffage/coupe (30), au moins un réflecteur supérieur (52) sur l'unité de chauffage (50) qui assure le chauffage des fils enroulés sur le tambour (21) lorsque ledit réflecteur supérieur (52) est aligné avec le réflecteur inférieur (51), dans lequel au moins un élément chauffant (53) est situé à l'intérieur du réflecteur supérieur (52).

2. La machine (M) selon la revendication 1, dans laquelle elle comprend au moins deux éléments d'enroulement (211) reliés à une plaque latérale (210) des deux côtés, formant des bords extérieurs pour permettre aux fils d'être enroulés sur le tambour (21) qui peut tourner sur son axe.

3. La machine (M) selon la revendication 1, dans laquelle le réflecteur inférieur (51) est positionné entre deux éléments d'enroulement successifs (211) dudit tambour (21) de telle sorte qu'il soit situé plus à l'intérieur que le bord extérieur desdits éléments d'enroulement successifs (211),
il comprend en outre plus de deux éléments d'enroulement (211) fixés entre les deux plaques latérales (210) de telle sorte que, lorsque le fil est enroulé sur le tambour (21) en fonction de la longueur du fil à couper, la longueur linéaire dans un tour autour de l'axe de rotation correspond à la longueur de fil souhaitée.

4. La machine (M) selon la revendication 1, comprenant un mécanisme de transfert de fil (40) avec un mécanisme de réglage de tension (44) qui permet à la tension du fil enroulé par rotation du tambour (21) sur son axe par une force mécanique.

5. La machine (M) selon la revendication 1, comprenant une glissière de transfert de fil (41) positionné parallèlement au tambour (21) pour garantir qu'une poulie (43) comprenant le fil à enrouler sur le tambour (21) soit alignée avec le tambour (21) et que le fil sur le tambour (21) soit orienté de manière à ne pas se chevaucher, et un chariot de transfert de fil (42) qui peut se déplacer linéairement sur ladite glissière de transfert de fil (41).

6. La machine (M) selon la revendication 1, comprenant un tambour (21) fixé à la plaque latérale (210) comprenant une ouverture de fixation du réflecteur inférieur (2101) positionnée dans la même direction que celle-ci afin de permettre la fixation du réflecteur inférieur (51).

7. La machine (M) selon la revendication 1, comprenant un élément d'axe de tambour (2103) positionné au centre d'au moins l'une des plaques latérales (210) pour permettre au tambour (21) de tourner autour d'un seul axe.

8. La machine (M) selon la revendication 1, comprenant au moins deux corps de réflecteur inférieurs (510) pour permettre au réflecteur inférieur (51) d'être fixé sur la plaque latérale (210).

9. La machine (M) selon la revendication 1, comprenant au moins un étrier de maintien du réflecteur inférieur (512) pour permettre au réflecteur inférieur (51) d'être fixé sur le corps du réflecteur inférieur (510).

10. La machine (M) selon la revendication 1, comprenant au moins deux étriers de tambour (22) qui permettent au tambour (21) d'être fixé sur le corps de machine (10) et d'être logé sur les éléments d'axe du tambour (2103).

11. La machine (M) selon la revendication 1, comprenant la coupeuse (31) qui permet de couper les fils à l'aide d'un couteau (311) qui se déplace linéairement sur au moins une glissière de manière à se déplacer parallèlement au tambour (21) positionné entre un corps latéral (300) du mécanisme de chauffage/coupe afin d'assurer la coupe des fils enroulés sur le tambour (21) le long du tambour (21).

12. La machine (M) selon la revendication 1, comprenant au moins un étrier de chauffage (531) qui permet de fixer le chauffage (53) dans le réflecteur supérieur (52).

13. La machine (M) selon la revendication 1, comprenant le réflecteur inférieur (51) avec un rayon intérieur (511) qui assure un durcissement rigide des fils par réflexion inverse des rayons réfléchis par le réflecteur supérieur (52) ou formant une distance pour empêcher le contact de la coupeuse (31) avec le réflecteur inférieur (51) pendant la coupe.

14. Une méthode pour empêcher l'effilochage en durcissant les extrémités des fils à couper aux dimensions souhaitées en enroulant les fils chirurgicaux sur au moins une forme cylindrique d'une machine (M) selon l'une quelconque des revendications 1-13 précédentes ; dans laquelle ledit fil est maintenu entre le réflecteur inférieur (51) et le réflecteur supérieur (52) en recouvrant les fils enroulés sur le tambour (21) ; dans lequel le réflecteur inférieur (51) est configuré pour être aligné exactement avec le réflecteur supérieur (52) de l'unité de chauffage (50) en faisant tourner le tambour (21) sur son axe ; dans lequel un processus de chauffage régional est effectué dans les fils par irradiation dans la zone fermée créée par le réflecteur supérieur (52) et le réflecteur inférieur (51) avec au moins un chauffage infrarouge dans ledit réflecteur supérieur (52).

15. La méthode selon la revendication 14, qui présente la structure de l'unité de chauffage (50) et du mécanisme de coupe (310) positionnés à des emplacements différents sur le mécanisme de chauffage/coupe (30) afin de déterminer la position de travail en tournant sur le même axe, et qui comprend les processus consistant à faire tourner le mécanisme de chauffage/coupe (30) sur son axe et à rapprocher le mécanisme de chauffage/coupe (30) du tambour (21) avec un mouvement linéaire afin d'assurer le rapprochement de l'unité concernée du tambour (21) en fonction de l'opération à effectuer sur le fil enroulé sur le tambour (21).
